# EUROPEAN PATENT SPECIFICATION

(11) **EP 1 232 755 B1**
(45) Date of publication and mention of the grant of the patent: **15.08.2007**
(21) Application number: 02003555.6
(22) Date of filing: 15.02.2002
(51) Int. Cl.: A61K 38/49, A61K 39/395, A61P 31/18

(54) **Anti-HIV agents**
Anti-HIV Substanz
Agent anti-vih

(30) Priority: 20.02.2001 JP 2001042655; 19.06.2001 JP 2001184284
(43) Date of publication of application: 21.08.2002
(73) Proprietor: JCR PHARMACEUTICALS Co., LTD., Ashiya, Hyogo 659-0021 (JP)
(72) Inventor: Wada, Manabu, Ashiya-shi, Hyogo 659-0041 (JP); Wada, Naokou, Ashiya-shi, Hyogo 659-0041 (JP)
(74) Representative: Behnisch, Werner

(56) References cited:
- WO-A-02/09753
- WO-A-96/13160
- WADA MANABU ET AL: "Amino-terminal fragment of urokinase-type plasminogen activator inhibits HIV-1 replication" BIOCHEMICAL AND BIOPHYSICAL RESEARCH COMMUNICATIONS, ACADEMIC PRESS, SAN DIEGO, CA, US, vol. 284, no. 2, 8 June 2001 (2001-06-08), pages 346-351, XP002183644 ISSN: 0006-291X

### SEQUENCE LISTING

<110> JCR Pharmaceuticals Co.,.Ltd.
<120> Anti-HIV agents
<130> GP47
<160> 5
<210> 1
   <211> 1296
   <212> DNA
   <213> Homo sapience
<400> 1
   atg aga gcc ctg ctg gcg cgc ctg ctt ctc tgc gtc ctg gtc gtg agc 48
   Met Arg Ala Leu Leu Ala Arg Leu Leu Leu Cys Val Leu Val Val Ser
   1 5 10 15
   gac tcc aaa ggc agc aat gaa ctt cat caa gtt cca tcg aac tgt gac 96
   Asp Ser Lys Gly Ser Asn Glu Leu His Gln Val Pro Ser Asn Cys Asp
   20 25 30
   tgt cta aat gga gga aca tgt gtg tcc aac aag tac ttc tcc aac att 144
   Cys Leu Asn Gly Gly Thr Cys Val Ser Asn Lys Tyr Phe Ser Asn Ile
   35 40 45
   cac tgg tgc aac tgc cca aag aaa ttc gga ggg cag cac tgt gaa ata 192
   His Trp Cys Asn Cys Pro Lys Lys Phe Gly Gly Gln His Cys Glu Ile
   50 55 60
   gat aag tca aaa acc tgc tat gag ggg aat ggt cac ttt tac cga gga 240
   Asp Lys Ser Lys Thr Cys Tyr Glu Gly Asn Gly His Phe Tyr Arg Gly
   65 70 75 80
   aag gcc agc act gac acc atg ggc cgg ccc tgc ctg ccc tgg aac tct 288
   Lys Ala Ser Thr Asp Thr Met Gly Arg Pro Cys Leu Pro Trp Asn Ser
   85 90 95
   gcc act gtc ctt cag caa acg tac cat gcc cac aga tct gat gct ctt 336
   Ala Thr Val Leu Gln Gln Thr Tyr His Ala His Arg Ser Asp Ala Leu
   100 105 110
   cag ctg ggc ctg ggg aaa cat aat tac tgc agg aac cca gac aac cgg 384
   Gln Leu Gly Leu Gly Lys His Asn Tyr Cys Arg Asn Pro Asp Asn Arg
   115 120 125
   agg cga ccc tgg tgc tat gtg cag gtg ggc cta aag ccg ctt gtc caa 432
   Arg Arg Pro Trp Cys Tyr Val Gln Val Gly Leu Lys Pro Leu Val Gln
   130 135 140
   gag tgc atg gtg cat gac tgc gca gat gga aaa aag ccc tcc tct cct 480
   Glu Cys Met Val His Asp Cys Ala Asp Gly Lys Lys Pro Ser Ser Pro
   145 150 155 160
   cca gaa gaa tta aaa ttt cag tgt ggc caa aag act ctg agg ccc cgc 528
   Pro Glu Glu Leu Lys Phe Gln Cys Gly Gln Lys Thr Leu Arg Pro Arg
   165 170 175
   ttt aag att att ggg gga gaa ttc acc acc atc gag aac cag ccc tgg 576
   Phe Lys Ile Ile Gly Gly Glu Phe Thr Thr Ile Glu Asn Gln Pro Trp
   180 185 190
   ttt gcg gcc atc tac agg agg cac cgg ggg ggc tct gtc acc tac gtg 624
   Phe Ala Ala Ile Tyr Arg Arg His Arg Gly Gly Ser Val Thr Tyr Val
   195 200 205
   tgt gga ggc agc ctc atc agc cct tgc tgg gtg atc agc gcc aca cac 672
   Cys Gly Gly Ser Leu Ile Ser Pro Cys Trp Val Ile Ser Ala Thr His
   210 215 220
   tgc ttc att gat tac cca aag aag gag gac tac atc gtc tac ctg ggt 720
   Cys Phe Ile Asp Tyr Pro Lys Lys Glu Asp Tyr Ile Val Tyr Leu Gly
   225 230 235 240
   cgc tca agg ctt aac tcc aac acg caa ggg gag atg aag ttt gag gtg 768
   Arg Ser Arg Leu Asn Ser Asn Thr Gln Gly Glu Met Lys Phe Glu Val
   245 250 255
   gaa aac cta atc cta cac aag gac tac agc gct gac acg ctt gct cac 816
   Glu Asn Leu Ile Leu His Lys Asp Tyr Ser Ala Asp Thr Leu Ala His
   260 265 270
   cac aac gac att gcc ttg ctg aag atc cgt tcc aag gag ggc agg tgt 864
   His Asn Asp Ile Ala Leu Leu Lys Ile Arg Ser Lys Glu Gly Arg Cys
   275 280 285
   gcg cag cca tcc cgg act ata cag acc atc tgc ctg ccc tcg atg tat 912
   Ala Gln Pro Ser Arg Thr Ile Gln Thr Ile Cys Leu Pro Ser Met Tyr
   290 295 300
   aac gat ccc cag ttt ggc aca agc tgt gag atc act ggc ttt gga aaa 960
   Asn Asp Pro Gln Phe Gly Thr Ser Cys Glu Ile Thr Gly Phe Gly Lys
   305 310 315 320
   gag aat tct acc gac tat ctc tat ccg gag cag ctg aaa atg act gtt 1008
   Glu Asn Ser Thr Asp Tyr Leu Tyr Pro Glu Gln Leu Lys Met Thr Val
   325 330 335
   gtg aag ctg att tcc cac cgg gag tgt cag cag ccc cac tac tac ggc 1056
   Val Lys Leu Ile Ser His Arg Glu Cys Gln Gln Pro His Tyr Tyr Gly
   340 345 350
   tct gaa gtc acc acc aaa atg ctg tgt gct gct gac cca cag tgg aaa 1104
   Ser Glu Val Thr Thr Lys Met Leu Cys Ala Ala Asp Pro Gln Trp Lys
   355 360 365
   aca gat tcc tgc cag gga gac tca ggg gga ccc ctc gtc tgt tcc ctc 1152
   Thr Asp Ser Cys Gln Gly Asp Ser Gly Gly Pro Leu Val Cys Ser Leu
   370 375 380
   caa ggc cgc atg act ttg act gga att gtg agc tgg ggc cgt gga tgt 1200
   Gln Gly Arg Met Thr Leu Thr Gly Ile Val Ser Trp Gly Arg Gly Cys
   385 390 395 400
   gcc ctg aag gac aag cca ggc gtc tac acg aga gtc tca cac ttc tta 1248
   Ala Leu Lys Asp Lys Pro Gly Val Tyr Thr Arg Val Ser His Phe Leu
   405 410 415
   ccc tgg atc cgc agt cac acc aag gaa gag aat ggc ctg gcc ctc tga 1296
   Pro Trp Ile Arg Ser His Thr Lys Glu Glu Asn Gly Leu Ala Leu
   420 425 430
<210> 2
   <211> 431
   <212> PTN
   <213> Homo sapience
<400> 2
   Met Arg Ala Leu Leu Ala Arg Leu Leu Leu Cys Val Leu Val Val Ser
   1 5 10 15
   Asp Ser Lys Gly Ser Asn Glu Leu His Gln Val Pro Ser Asn Cys Asp
   20 25 30
   Cys Leu Asn Gly Gly Thr Cys Val Ser Asn Lys Tyr Phe Ser Asn Ile
   35 40 45
   His Trp Cys Asn Cys Pro Lys Lys Phe Gly Gly Gln His Cys Glu Ile
   50 55 60
   Asp Lys Ser Lys Thr Cys Tyr Glu Gly Asn Gly His Phe Tyr Arg Gly
   65 70 75 80
   Lys Ala Ser Thr Asp Thr Met Gly Arg Pro Cys Leu Pro Trp Asn Ser
   85 90 95
   Ala Thr Val Leu Gln Gln Thr Tyr His Ala His Arg Ser Asp Ala Leu
   100 105 110
   Gln Leu Gly Leu Gly Lys His Asn Tyr Cys Arg Asn Pro Asp Asn Arg
   115 120 125
   Arg Arg Pro Trp Cys Tyr Val Gln Val Gly Leu Lys Pro Leu Val Gln
   130 135 140
   Glu Cys Met Val His Asp Cys Ala Asp Gly Lys Lys Pro Ser Ser Pro
   145 150 155 160
   Pro Glu Glu Leu Lys Phe Gln Cys Gly Gln Lys Thr Leu Arg Pro Arg
   165 170 175
   Phe Lys Ile Ile Gly Gly Glu Phe Thr Thr Ile Glu Asn Gln Pro Trp
   180 185 190
   Phe Ala Ala Ile Tyr Arg Arg His Arg Gly Gly Ser Val Thr Tyr Val
   195 200 205
   Cys Gly Gly Ser Leu Ile Ser Pro Cys Trp Val Ile Ser Ala Thr His
   210 215 220
   Cys Phe Ile Asp Tyr Pro Lys Lys Glu Asp Tyr Ile Val Tyr Leu Gly
   225 230 235 240
   Arg Ser Arg Leu Asn Ser Asn Thr Gln Gly Glu Met Lys Phe Glu Val
   245 250 255
   Glu Asn Leu Ile Leu His Lys Asp Tyr Ser Ala Asp Thr Leu Ala His
   260 265 270
   His Asn Asp Ile Ala Leu Leu Lys Ile Arg Ser Lys Glu Gly Arg Cys
   275 280 285
   Ala Gln Pro Ser Arg Thr Ile Gln Thr Ile Cys Leu Pro Ser Met Tyr
   290 295 300
   Asn Asp Pro Gln Phe Gly Thr Ser Cys Glu Ile Thr Gly Phe Gly Lys
   305 310 315 320
   Glu Asn Ser Thr Asp Tyr Leu Tyr Pro Glu Gln Leu Lys Met Thr Val
   325 330 335
   Val Lys Leu Ile Ser His Arg Glu Cys Gln Gln Pro His Tyr Tyr Gly
   340 345 350
   Ser Glu Val Thr Thr Lys Met Leu Cys Ala Ala Asp Pro Gln Trp Lys
   355 360 365
   Thr Asp Ser Cys Gln Gly Asp Ser Gly Gly Pro Leu Val Cys Ser Leu
   370 375 380
   Gln Gly Arg Met Thr Leu Thr Gly Ile Val Ser Trp Gly Arg Gly Cys
   385 390 395 400
   Ala Leu Lys Asp Lys Pro Gly Val Tyr Thr Arg Val Ser His Phe Leu
   405 410 415
   Pro Trp Ile Arg Ser His Thr Lys Glu Glu Asn Gly Leu Ala Leu
   420 425 430
<210> 3
   <211> 4
   <212> PTN
   <213> Homo sapience
<400> 3
   Lys Lys Phe Gly
<210> 4
   <211> 12
   <212> PTN
   <213> Homo sapience
<400> 4
   Ala Ser Thr Asp Thr Met Gly Arg Pro Cys Leu Pro
   5 10
<210> 5
   <211> 10
   <212> PTN
   <213> Homo sapience
<400> 5
   Arg Arg Pro Trp Cys Tyr Val Gln Val Gln
   5 10

## Description

### FIELD OF THE INVENTION

The present invention relates to a screening method for anti-HIV agents, and the use of determined ligand molecules binding to CD87 for the manufacture of pharmaceutical agents agents for inhibiting reproduction of HIV in an individual infected with the virus.

### BACKGROUND OF THE INVENTION

HIV, the virus causing acquired immunodeficiency syndrome (AIDS), is an RNA virus that belongs to *Lentivirus* of *Retroviridae* family. Infection and reproduction of HIV takes place in the following manner. At first, an envelope protein of the HIV particle, gp120 (glycoprotein 120), binds to CD4 on the surface of target cells. Thus bound Gp120 and CD4 then bind to a chemokine receptor (primarily, CCR5 on macrophages or CXCR4 on T cells), which serves as a co-receptor, to form a complex consisting of gp120, CD4 and the chemokine receptor. This is followed by binding of another envelope protein, gp41, to the plasma membrane of the target cell. This leads to fusion of the envelope with the cell membrane, and the core of the virus thereby enters the cell. Once in the cell, HIV is uncoated and a double-stranded provirus DNA is synthesized using the template RNA genome by reverse transcriptase brought in by the virus. The provirus DNA then integrate into the host cell chromosomal DNA with the help of integrase, which also is a viral enzyme. Using the LTR (long terminal repeat) at the 5' end of the provirus as a promoter, transcription of the incorporated provirus gives viral mRNA. Several mRNAs of different length are produced in this process, which are divided into two groups, i.e., mRNAs for synthesis of viral proteins and one used as the viral genomic RNA. The viral proteins include, for example, structural proteins for forming viral particles, and proteins for accelerating replication of the virus. For example, a viral protein Tat binds to the 5' LTR region of the provirus incorporated in the host's genome and by so doing increases production of viral RNA transcript as much as several hundredfold. On the other hand, the structural proteins for forming viral particles associate with the viral genome RNA within the cell, near the cell membrane, to assemble viral particles. The viral particles thus assembled are then released out of the cell through budding. The mechanisms of assembling and budding are still not well known. In the released particles, a protease is activated, processing takes place, and this gives mature, infectious viral particles. HIV rapidly reproduces by repeating the whole process consisting of binding to CD4 on the target cells, integration into the host's chromosomal DNA, replication, budding and maturation. Along with reproduction of HIV, destruction of host's CD4-positive cells takes place. To cope with this, the host induces rapid propagation of fresh CD4-positive cells to fill up the loss. The dynamic equilibrium provided by this will last several years after infection, but sooner or later the supply of CD4-positive cells will become unable to catch up with the loss, resulting in a total breakdown of the immune system and occurrence of various symptoms of AIDS.

In an individual infected with HIV, a variety of immune reactions occur in order to get rid of HIV. Among them are, for example, production of neutralizing antibodies to the viral antigens, and elimination of infected cells by cytotoxic T cells. It is also known that some humoral factors produced by CD8-positive cells play important roles in keeping HIV-infected individuals to stay within the symptom-free period [Levy, J.A., et al., Immunol. Today, 17: 217-224(1996), Fauci, A.S., Nature, 384: 529-534(1996)]. Among such factors, chemokines (RANTES, MIP-α, 1 β, SDF-1) and IL-16 have been identified so far. They, however, do not provide sufficient basis needed for fully explaining the anti-HIV activity derived from CD8-positive cells, suggesting involvement of some unidentified HIV-suppressive factors.

Chemokines act to inhibit HIV from entering target cells (macrophages and T cells). On the other hand, there are also reports suggesting that chemokines accelerate HIV replication in macrophages. IL-16 is known to suppress the transcription process of HIV, but a very high concentration of it is thought to be required to exhibit any such effect. Though efforts have been made to bring these compounds under development as therapeutics for AIDS, none of them has reached the stage of practical application. Some of unidentified anti-HIV factors, on the other hand, are expected to inhibit the transcription process of HIV. However, as long as such factors remain unidentified, any of their mechanism of action has been staying just a matter of speculation.

Summarized below are inhibition rates of HIV reproduction determined with factors of organismic origin that have so far been reported to have inhibitory activity on HIV.
(1) RANTES (MW = 7,851): In a system employing PM1 cells and HIV-1_{BaL} strain; 5 % at 0.78 ng/mL (= 0.1 nM), 50 % at 1.56 ng/mL (= 0.2 nM), and 90 % at 3.12 ng/mL (= 0.4 nM) [Cocchi, F. et al., Science 270:1811-1815(1995)].
(2) MIP-a (MW = 7,717): In the same system as used for RANTES above; 0 % at 3.12 ng/mL, 5 % at 6.25 ng/mL (= 0.8 nM), and 50 % at 12.5 ng/mL (= 1.6 nM)[Cocchi, F. et al., supra].
(3) MIP-1 β (MW = 7,819): In the same system as used for RANTES above; 0 % at 0.78 ng/mL, 5 % at 1.56 ng/mL (= 0.2 nM), 15 % at 3.12 ng/mL (= 0.4 nM), and 60 % at 6.25 ng/mL (= 0.8 nM)[Cocchi, F. et al., supra).
(4) IL-16 (MW = 12,422): 61 % at 40-70 ng/mL ( ~1 nM) and 76 % at 400~700 ng/mL (= 10 nM), respectively for active tetramer, and 50 % at 20 µg/mL for monomer [Baier, M. et al., Nature 378:563(1995), Amiel, C. et al., J. Infect. Dis. 179:83-91(1999)].
(5) MDC (MW = 7,936): 20 % at 25 ng/mL (= 3.15 nM), 50 % at 50 ng/mL (= 6.3 nM), and 78 % at 200 ng/mL (= 25 nM)[ Pal, R. et al., Science 278:695-698(1997)].
(6) SDF-1 (MW = 8,698): 30 % at 500 ng/mL (=57.5 nM) and 60 % at 1 µg/mL (= 115 nM), respectively, when measured as inhibition rate of virus penetration, and 80-85 % at 700 µg/mL (= 80.5 nM) when measured as inhibition rate of reproduction [Oberlin, E. et al., Nature 382:833-835(1996)].

Reverse transcriptase inhibitors (which inhibit provirus formation) and protease inhibitors (which inhibit maturation of the viral particles) now have found practical application as AIDS therapeutics. Thus, six nucleoside-based reverse transcriptase inhibitors, two non-nucleoside-based reverse transcriptase inhibitors and five protease inhibitors are now commercially available. Three-drug combination therapy (HAART: highly active antiretroviral therapy) employing a combination of three of those drugs (in general, two reverse transcriptase inhibitors and one protease inhibitor) has become available, making it possible to lower blood virus levels below detection limit.

However, even such a three-drugs combination therapy can not completely eliminate HIV from infected individuals. Therefore, in order to prevent development of AIDS, those infected with HIV have to keep taking those drugs throughout their lives. To be effective, those drugs must be taken in large amounts, and the time schedule for taking each of them will be rigidly fixed. These sometimes make it difficult to take them following predetermined schedules, thereby resulting in poor compliance and reduced therapeutic effects. Moreover, it is not unusual for those drugs to cause severe side effects.

WO-A-96/13160 discloses an in vitro method of inhibiting the infectivity of Human Immunodeficiency Virus (HIV) in a liquid that may contain HIV by exposing the liquid to a urokinase-type plasminogen activator at a concentration and for a time sufficient to inactivate HIV in the liquid. In particular, it teaches an anti-HIV activity of HMW-uPA, wherein the uPA is defined as containing at least the catalytic domain of the B chain. However, it can not be derived from WO-A-96/13160 that amino acids of the A chain have to be present as an essential element in order to achieve anti-HIV activity.

On the other hand, mutation takes place quite frequently in HIV. A resistant strain of virus will thus emerge within several months, in particular under treatment with a single drug. And, resistant virus rapidly reproduces when a drug treatment is interrupted, making the drug ineffective even if the same treatment is resumed. Furthermore, virus that has become resistant to a drug often acquires multidrug resistance also to other anti-HIV drugs that act by the same mechanism of action. Therefore, it is critical to prevent emergence of resistant HIV in order to keep AIDS from developing as well as for its treatment. For this purpose, it is important to simultaneously suppress HIV at more than one stages of its life cycle. Thus, new types of drugs are needed that inhibit HIV reproduction at a different stage from those which the presently used anti-HIV drugs act on. In this respect, some humoral factors produced by CD8-positive cells are expected to be potential compounds for new AIDS therapeutics because those factors, although with unknown mechanism, play a significant role in suppressing HIV reproduction.

If a severe side effect or resistant HIV has appeared during AIDS treatment, it becomes necessary to change the drugs to be administered. However, there is only a poor choice at present. Thus, there is a need for anti-HIV drugs acting by a different mechanism of action from those known with conventional drugs, in order for widening a choice of AIDS therapeutics and avoiding the problems of resistant HIV.

In this situation, the objective of the present invention is to provide a new type of anti-HIV agent that acts by a mechanism of action different from those already being clinically used or under development.

### SUMMARY OF THE INVENTION

The present inventors isolated CD8-positive cells from a human infected with HIV and immortalized and cloned them making use of HTLV-1, and then purified an unknown factor that exhibited anti-HIV activity in the supernatant of the cells, and examined its structure and functions. As a result, it was revealed that the factor was the amino-terminal fragment (ATF: amino-terminal fragment) of the high molecular weight urokinase-type plasminogen activator. It was also found: (1) that the factor exhibited anti-HIV activity at a surprisingly low concentration (0.74 ng/mL), (2) that it was effective on both macrophage-tropic and T cell-tropic strains of HIV, and (3) that it was likely that the factor suppressed later stages than the stage of translation of viral mRNA in the HIV life cycle, in particular the stages of assembling of viral particles or budding. While ATF has a property of specifically binding to CD87 on the surface of cells, it was also found using healthy human urine urokinase, that the high molecular weight urokinase-type plasminogen activator (HMW-uPA), which had been known to include ATF moiety at an end of its molecule and to be a ligand molecule to CD87, also had anti-HIV activity. In addition, it was confirmed that ATF obtained by decomposing healthy human urine urokinase also had anti-HIV activity. Moreover, it was found that anti-CD87 antibody had an ATF-like anti-HIV activity, and that the anti-HIV activity of ATF was mediated by the same target molecule as the anti-CD87 antibody's target (i.e., CD87). These findings made it clear that it is possible to suppress reproduction of HIV by blocking CD87 by bringing CD87 on potential HIV host cells into contact with one of specifically binding ligand molecule such as ATF, HMW-uPA or fragments thereof or analogues thereto.

The present invention is directed to a screening method as defined in claim 1 and to the use of a ligand molecule binding to CD84 for the manufacture of a pharmaceutical composition for suppression of reproduction of HIV in a human infected with HIV, wherein the ligand molecule is one as defined in claims 2-6.

Thus the present invention describes an anti-HIV agent comprising as an active component a ligand molecule binding to CD87. The ligand molecule may be a fragment of the high molecular weight urokinase-type plasminogen activator insofar as the fragment has a specific binding affinity to CD87. Furthermore, the ligand molecule may be the amino-terminal fragment (ATF) of the high molecular weight urokinase-type plasminogen activator, as well as a fragment of ATF having a specific binding affinity to CD87. Other examples of the ligand molecule include an anti-CD87 antibody (monoclonal or polyclonal), as well as a fragment of an anti-CD87 antibody having a specific binding affinity to CD87.

The present invention also describes a pharmaceutical composition comprising, as an active component, a ligand molecule binding to CD87. Examples of such ligand molecules are as mentioned above. Among such ligand molecules, ATF and fragments thereof having a specific binding affinity to CD87 are especially preferred.

The present invention is directed to a method for screening for an anti-HIV agent comprising separately bringing compounds to be tested into contact with CD87 and selecting from the compounds a compound that specifically binds to CD87.

The present invention further describes a method for treating an HIV-infected human for suppression of reproduction of HIV in the human comprising administering to the human an HIV reproduction-suppressive amount of a ligand molecule binding to CD87. Examples of such ligand molecules are as mentioned above.

The present invention further is directed to the use of a ligand molecule binding to CD87 for the manufacture of a pharmaceutical composition for suppression of reproduction of HIV in a human infected with HIV, wherein the ligand molecule binding to CD87 is a fragment of or an analogue to the high molecular weight urokinase-type plasminogen activator, wherein the fragment comprises amino acids 21-155 of the prepro-urokinase (sc-uPA) and does not extend beyond amino acid 178 of the sc-uPA. Examples of such ligand molecules are as mentioned above.

### BRIEF DESCRIPTION OF THE FIGURES

Fig. 1 illustrates the primary structures of the urokinase-type plasminogen activator and ATF.
Fig. 2 illustrates the structure of pSEAP-Basic.
Fig. 3 illustrates the structure of pREP7.
Fig. 4 illustrates the structure of pSBR.
Fig. 5 illustrates the structure of pNL4-3 and the region amplified by PCR.
Fig. 6 illustrates the structure of pSBR-HIV.
Fig. 7 is a graph illustrating anti-HIV activity of the eluate fractions from a hydroxyapatite column and the protein concentration corresponding to the fractions.
Fig. 8 is a SDS/PAGE electropherogram of the eluate fractions from a hydroxyapatite column.
Fig. 9 is a graph illustrating anti-HIV activity of the eluate fractions from a HiPrep Sephacryl S-100 column loaded with a human urine urokinase bulk material and the protein concentration corresponding to the fractions.
Fig. 10 is a SDS/PAGE electropherogram of the eluate fractions from a HiPrep Sephacryl S-100 column loaded with a human urine urokinase bulk material.
Fig. 11 is a graph illustrating the result of an anti-HIV activity assay (p17) in co-culture (T cell lines).
Fig. 12 is a graph illustrating the result of an anti-HIV activity assay (p 17) in co-culture (macrophage lines).
Fig. 13 is a graph illustrating the result of a SEAP reporter assay in the culture of non-infected cells (MC141).
Fig. 14 is a graph illustrating the result of a SEAP reporter assay in the culture of non-infected cells (CL35).
Fig. 15 is a graph illustrating the result of a temporary transfection assay with infectious HIV-DNA.
Fig. 16 is a graph illustrating the result of a solo-culture assay of chronically infected cells (U1).
Fig. 17 is a graph illustrating the profile of HIV amount over the days following acute infection.
Fig. 18 is a group of graphs separately illustrating the suppression of viral reproduction by ATF on different days after infection.
Fig. 19 is a graph illustrating the effect of anti-CD87 antibody on ATF's anti-HIV activity in T cell lines.
Fig. 20 is a graph illustrating the effect of anti-CD87 antibody on ATF's anti-HIV activity in macrophage lines

### DETAILED DESCRIPTION OF THE INVENTION

CD87, one of the CD antigens, is a membrane protein without an intra-cellular domain and belongs to a GPI (glycosylphosphatidylinositol) anchor-type family. It is expressed on the surface of cells such as T cells and monocytes (including macrophages). It is known that this protein has high affinity to pro-urokinase as well as to the high molecular weight urokinase-type plasminogen activator, and that it serves as a receptor on the surface of such cells as T cells and monocytes. Human CD87 is synthesized at first in a prepro-form consisting of amino acids 1-335, from which the signal peptide moiety (amino acids 1-22) and then the carboxyl terminal amino acids (306-355) are cleaved through processing. To the carboxyl terminus (305 Gly) thus created is added a glycolipid (GPI), through which the protein is fixed to the cell membrane. In CD87, it is the N-terminal domain 1 (amino acids 1-92 of CD87) that is playing a main role in binding to ligand molecules such as pro-urokinase and the high molecular weight urokinase-type plasminogen activator [Seki et al, Seikagaku, 71(5): 350-352 (1999)].

HIV is divided into two subtypes, HIV-1 and HIV-2. Both HIV-1 and HIV-2 are a type of virus that is released from the host by budding, and they are genetically nearly indistinguishable. They share a common life cycle and reproduce in the same manner. Therefore, there is no need for distinguishing them from each other in the context of anti-HIV drugs, and actually they are in general viewed as being equivalent in the treatment with conventional anti-HIV drugs. In the present specification, the term "HIV" includes both "HIV-1" and "HIV-2" unless otherwise mentioned.

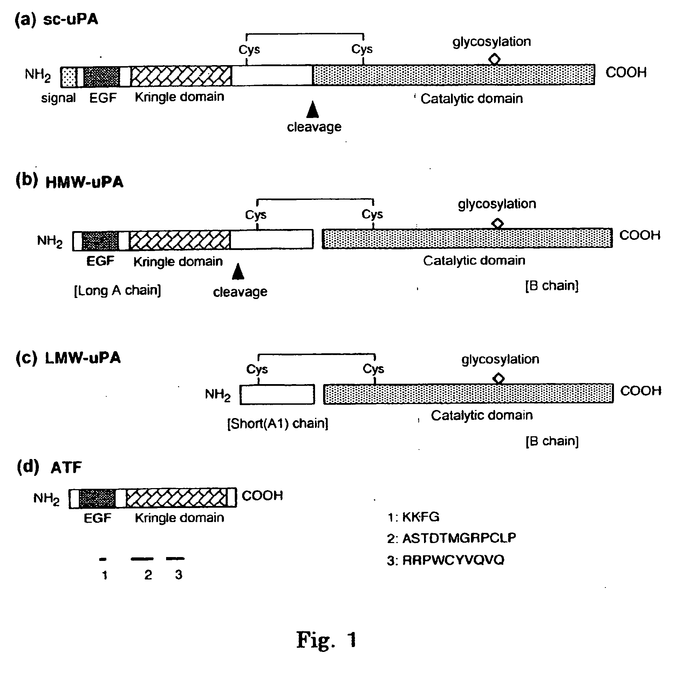

The high molecular weight urokinase-type plasminogen activator (HMW-uPA)(Fig. 1(b); amino acids 21-178 + amino acids 179-431) is a protein consisting of two peptide chains linked by a disulfide bond. The chains, long A and B, are formed by enzymatic cleavage (with plasmin, kallikrein, cathepsin, etc.) between amino acids 178 and 179 of pro-urokinase, which is formed by removal of the N-terminal signal peptide (amino acids 1-20) from a single chain protein called prepro-urokinase (sc-uPA)(Fig. 1(a); amino acids 1-431). HMW-uPA includes an EGF-like domain, a Kringle domain and a urokinase receptor (CD87) binding domain.

HMW-uPA then is cleaved between amino acids 155 and 156 in vivo, thereby giving rise to the low molecular weight urokinase-type plasminogen activator (LMW-uPA)(Fig. 1(c); amino acids 156-178 and amino acids 179-431) and the amino-terminal fragment (ATF)(Fig. 1(d); amino acids 21-155) that has no plasminogen activator activity. Cleavage between amino acids 155 and 156 also takes place during incubation in, e.g., a phosphate buffer solution, pH 8. Thus, ATF can be produced by simple incubation of HMW-uPA in a buffer solution (25-37°C). ATF includes the EGF-like domain, the Kringle domain and the urokinase receptor (CD87) binding domain of HMW-uPA in their entirety. In the Sequence Listing, the nucleotide sequence encoding sc-uPA and its amino acid sequence are set forth as SEQ ID NO:1 and NO:2, respectively. In the sequences set forth as SEQ ID NO:1 and NO:2, amino acids 1-20 correspond to the signal peptide, amino acids 21-431 pro-urokinase, amino acids 21-431 (with a cleavage between amino acids 178 and 179) HMW-uPA, amino acids 21-155 ATF, and amino acids 156-431 (with a cleavage between amino acids 178 and 179) LMW-uPA, respectively.

Transmission of HIV between humans is caused by macrophage-tropic HIV. With a lapse of time after infection, T cell-tropic HIV emerges in those who infected, which is considered to be a factor relating to a bad prognosis. CD87 occurs on both T cells and macrophages and ATF suppresses HIV reproduction in both of HIV-infected T cells and macrophages by suppressing release of HIV from those cells. This means that ATF will effectively work as an anti-HIV agent irrespective of the lapse of time after infection. In addition, ATF is effective even at a very low concentration (0.74 ng/mL). Therefore, the amount of ATF to be administered to a patient will be smaller than that of conventional anti-HIV drugs. This could create less physical burden on patients caused by administering the agent. HMW-uPA, which includes ATF at its N terminus, also has anti-HIV activity, although somewhat weaker than the latter, and can be used in the same manner as ATF. Once administered to an infected patient, HMW-uPA is expected to exhibit anti-HIV activity not only as it's intact molecule but also in the form of ATF generated by its cleavage in the body. On the other hand, anti-CD87 antibody, which suppresses HIV reproduction in HIV-infected T cells, is useful for suppressing HIV reproduction after the emergence of T cell-tropic HIV after infection.

The test results described below indicate that ATF, which is one of the active components of the anti-HIV agent of the present invention, suppresses the viral life cycle at later stages than that of translation of viral mRNA, in particular the stages of assembling of viral particles or budding, a different mechanism of action from those known with other anti-HIV drugs. ATF does not affect the growth of host cells, thus exhibiting no signs of cytotoxicity. Therefore, ATF used in combination with conventional anti-HIV drugs will shift the dynamic equilibrium of HIV reproduction in an infected patient in the direction in favor of the latter, and at the same time make it easier to avoid the emergence of resistant virus and problems of side effects, thus providing improved therapy for AIDS.

### <Naturally Occurring ATF and Recombinant ATF>

In the present invention, ATF may be prepared, for example, from the urokinase-type plasminogen activator obtained from healthy human urine [Stoppelli, P.M. et al., Proc. Natl. Acad. Sci. USA, 82:4939-4943 (1985)]. For example, HMW-uPA is incubated in 50 mM phosphate buffer, pH 8, containing 0.2 M sodium chloride for about 8 hours or more, and the reaction products are subjected to gel filtration (e.g., Sephadex G-100); ATF is obtained by separating eluate fractions corresponding to the last peak (peak 3: ATF) on the UV absorption curve from the fractions corresponding to preceding peaks, i.e., peak 1 (HMW-uPA) and peak 2 (LMW-uPA). Further purification may be performed by subjecting the ATF-containing fractions to ion-exchange chromatography (e.g., Mono S HR5/5 column; 50 mM sodium acetate buffer, pH 4.8, with a sodium chloride gradient of 0-1.0 M).

ATF may also be produced as a recombinant peptide by incorporating ATF-encoding DNA into a proper expression vector and then transforming a proper host cells (e.g., *E. coli,* yeast or mammalian cells) with the vector. As naturally occurring ATF has no sugar chains, a recombinant ATF produced by cells transformed using cDNA encoding naturally occurring ATF is of the same structure, and therefore has the same activity, as naturally occurring ATF. Fragments of ATF or HMW-uPA having ability of specifically binding to CD87 can be prepared, for example, by partial modification of ATF or HMW-uPA, for example by deletion of one or more amino acid residues from a terminus of their molecules,

### <Method for Screening Ligand Molecules>

The method of the present invention for screening for an anti-HIV agent, which comprises separately bringing compounds to be tested into contact with CD87 and selecting from the compounds a compound that specifically binds to CD87, can be performed using cells carrying CD87 on their surface (T cell strains, macrophage strains). Specific binding of a tested compound to CD87 can be detected by applying to CD87 any of desirable method known in the art for detection of specific binding of ligand molecules to their receptors. For example, a compound to be tested is mixed with CD87 produced by recombinant techniques, incubated, and then subjected to immune precipitation with anti-CD87 antibody. By detecting co-precipitation of the compound, occurrence of specific binding can be assessed.

The anti-HIV agent of the present invention can be administered to those infected with HIV through a parenteral route such as injection or implantation, as well as by transnasal or transpulmonary application. When the anti-HIV agent of the present invention is prepared in the form of injection, it may be provided as a preparation adapted to, for example, intravenous, intraperitoneal, intramuscular or subcutaneous injection. For injection, the anti-HIV agent of the present invention may be provided in the form of sterile, aqueous or non-aqueous, solution, suspension or emulsion. Examples of aqueous mediums include water and aqueous solution of one or more pharmaceutically acceptable inert solutes, e.g., salts, polysaccharides or polyalcohols. They may be adjusted to a pH value within a proper range with pharmaceutically acceptable buffers. Examples of such aqueous mediums include, but are not limited to, sodium chloride solution, Ringer-glucose solution, glucose solution, and lactate Ringer solution. In order to improve stability during storage of the preparation, it may be lyophilized.

As for a non-aqueous medium for an injectable preparation, one may use as desired any of non-aqueous mediums conventionally used in parenteral application, for example polyalcohols such as glycerol and propylene glycol, polyethylene glycol, vegetable oils (e.g., olive oil, soybean oil, rapeseed oil), organic esters such as ethyl oleate.

When providing the anti-HIV agent of the present invention in the form of an implant, any carrier for sustained release may be used which establishes prolonged release of the employed CD87 ligand molecule such as ATF. It is preferable to use such a carrier for it would lead to reduction of frequency and/or dose of the ligand molecule administered, to ease of handling, and to enhanced or prolonged effect. Examples of such carriers include, but are not limited to, liposomes, microspheres, and microcapsules made of natural or synthetic polymers. Further, examples of carriers suitable for prolonged and delayed release in most environment include gelatin, gum arabic, xanthan polymer, polylactic acid, polyglycolic acid, and lactate/polyglycolate copolymer.

Transnasal or transpulmonary application (inhalation) is a particularly effective way of administration for reducing patient's burden coming from administration of drugs. For transnasal or transpulmonary application (inhalation), the anti-HIV agent of the present invention may be in any form adapted to spraying and inhalation as fine particles, such as solution or powder. Examples of such preparations include a dry powder consisting of the mixture of a CD87 ligand molecule such as ATF and a carrier and having particle diameter of or less than 10 µm. Examples of such carriers used for this purpose include monosaccharides such as glucose and fructose, disaccharides such as lactose, maltose and sucrose, polysaccharides such as starch, cellulose, hyaluronic acid, chitin and chitosan, sugar alcohols such as sorbitol and mannitol, organic binders such as cellulose derivatives, e.g., hydroxypropylcellulose, hydroxypropylmethylcellulose, methylcellulose and hydroxyethylcellulose, as well as polyvinylpyrrolidone and polyvinyl alcohol, nonionic surfactants, proteins such as gelatin and casein, and synthetic polymers such as polyethylene glycol. Another example of preparation for transnasal or transpulmonary application is a CD87 ligand molecule, e.g., dry ATF, suspended in fluorocarbon propellant.

In the present invention, the dose of the active component of the anti-HIV agent per body weight of an infected patient is approximately 10 µg - 10 mg/kg/day for ATF, 10 µg - 10 mg/kg/day for HMW-uPA, and 10 µg - 10 mg/kg/day for an anti-CD87 antibody.

### EXAMPLES

The present invention will be described in further detail below with reference to working examples. However, it is not intended that the present invention be limited by the examples.

Listed below are the materials and methods employed for purification, identification of ATF and for determination of anti-HIV activity of ATF, HMW-uPA and anti-CD87 antibody.

### <Materials>

### 1) Plasmids

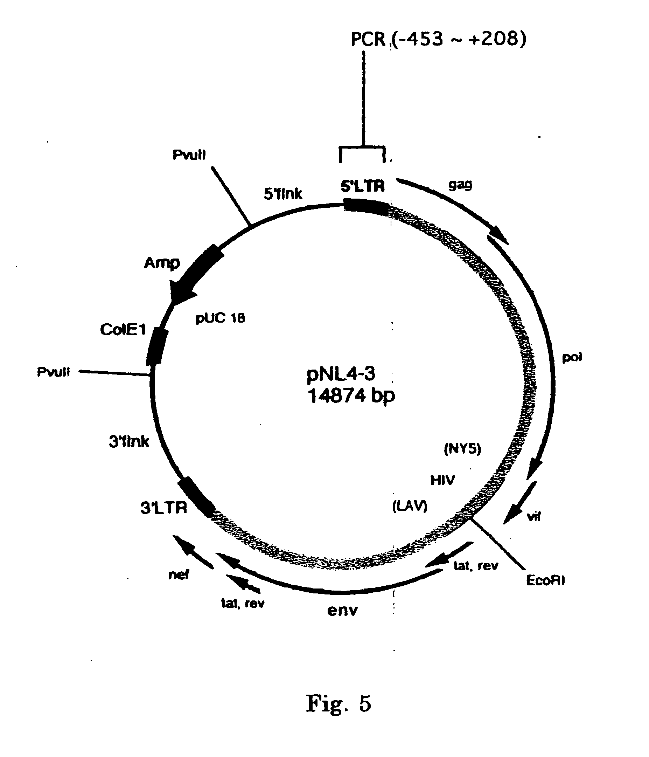

(i) pNL4-3: The plasmid that is deposited with NIH AIDS Research and Reference Reagent Program, catalog No. 114, was used (Fig. 5). This is a plasmid constructed by incorporating HIV-1 proviral genomic DNA isolated from the genome of an HIV-1-infected person into pUC18 plasmid vector [Adachi, A. et al., J. Virol., 59(2):284-291(1986)]. Transfection of a cell with this plasmid will cause the cell to produce infectious HIV-1 virus.
(ii) pSBR-HIV: This is a plasmid constructed by incorporating as a promoter the LTR region of pNL4-3 into the basic plasmid of pSEAP-Basic (CLONTECH, Palo Alto, CA, USA) at a location upstream of its reporter gene, i.e., the secretion-form alkaline phosphatase (SEAP) gene, and further incorporating a hygromycin resistance gene as a selection marker.

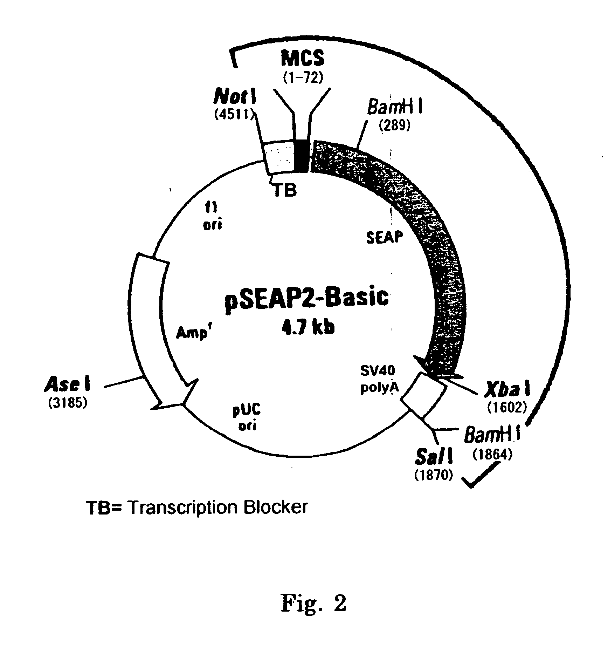

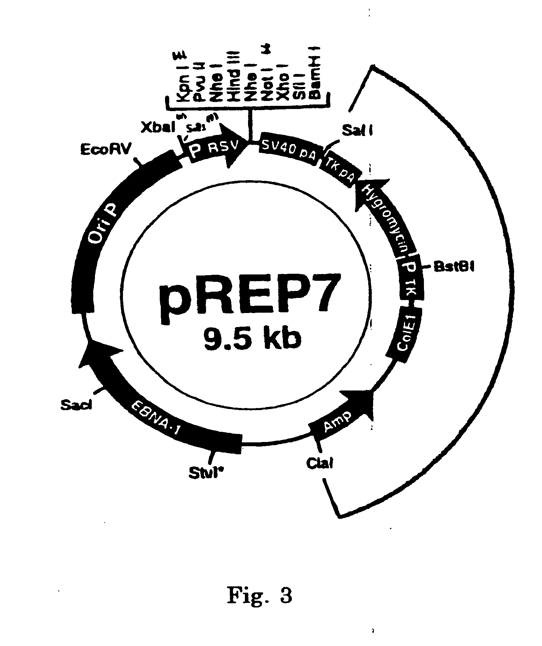

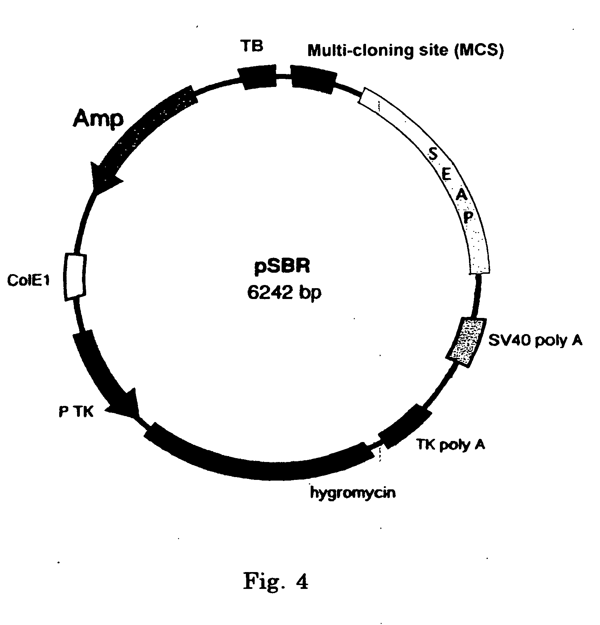

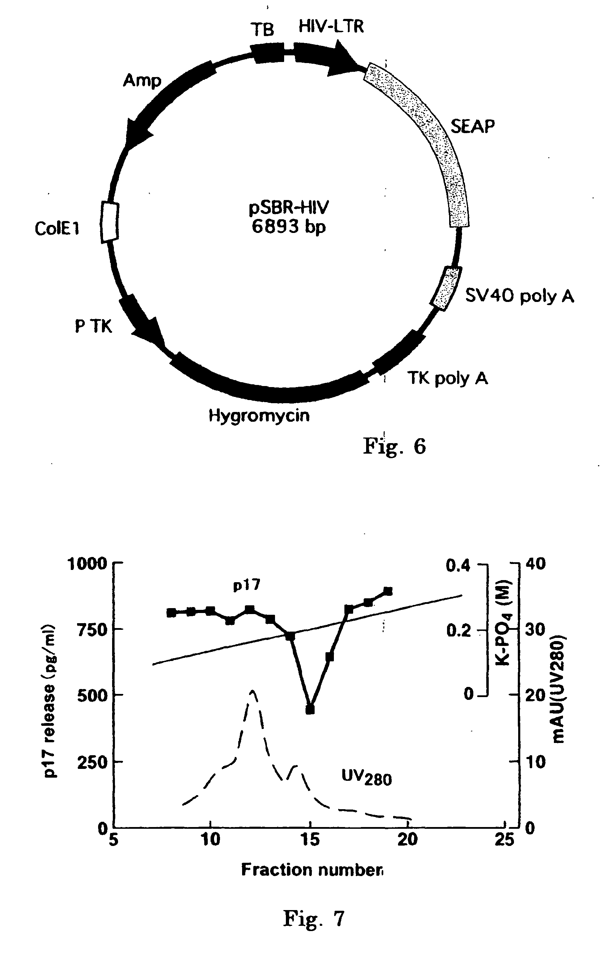

This plasmid was prepared through the following process of:
(a) digesting pSEAP-Basic (Fig. 2)(CLONTECH) with NotI and SalI to cut out a region (Fig. 2, indicated by the arc) including SEAP gene, and blunt-ending its NotI site with T4 polymerase,
(b) separately, digesting pREP7 (Fig. 3)(INVITROGEN, 9704CH, Groningen, the Netherlands) with SalI and ClaI to cut out a region (Fig. 3, indicated by the arc) including hygromycin resistance gene (Hygromycin), ColE1 and ampicillin resistance gene (Amp), and blunt-ended its ClaI site with T4 polymerase,
(c) ligating the fragment obtained in (a) above with the fragment obtained in (b) above to construct pSBR (Fig. 4),
(d) PCR-amplifying HIV-LTR from pNL4-3 using primers with an attached Xhol or HindIII site, respectively (Fig. 5), and digesting the PCR product with XhoI and HindIII, and
(e) digesting pSBR obtained in (c) above with HindIII and then inserting into this digestion product HIV-LTR obtained in (d) above to construct pSBR-HIV (Fig. 6).

In the above, pSBR-HIV was constructed as an example of a type of plasmids having HIV-LTR as a promoter and expressing a reporter gene (SEAP in the example). Any other plasmid may be constructed and used likewise that has HIV-LTR as a promoter and is able to express a suitable reporter gene.

### 2) Cells

(i) HUT.78: The cells deposited with NIH AIDS Research and Reference Reagent Program, Catalog No. 89, were used. This is a CD4-positive T cell line established from peripheral blood of a patient with Sezary syndrome, a chronic cutaneous lymphoma. The cells were cultured in RPMI1640 medium containing 10 % FCS (Gibco/BRL).
(ii) U937: The cells deposited with ATCC (American Type Culture Collection), catalog No. CRL-1593.2, and also with the National Institute of Health Sciences (Japan), JCRB Catalog No. 9021, were used. This is a CD4-positive monoblast line established from ascites of a patient with true histiocytic lymphoma. The cells were cultured in RPMI1640 medium containing 10 % FCS.
(iii) TALL-1: The cells deposited with the National Institute of Health Sciences (Japan), JCRB Catalog No. 0086, were purchased. This is a CD4-positive T cell line established from peripheral blood of a patient with acute lymphocytic leukemia. The cells were cultured in RPMI1640 medium containing 10 % FCS.
(iv) T4/NL4-3: This cell line was created by transfecting TALL-1 cells with pNL4-3. This is a cell line chronically infected with HIV-1. The cells were cultured in RPMI1640 medium containing 10 % FCS and 5 µM AZT.
(v) U1: The cells deposited with NIH AIDS Research and Reference Reagent Program, Catalog No. 165, were purchased. This is a cell line chronically infected with macrophage-tropic HIV-1 created by infecting U937 cells with a HIV-1 strain clinically isolated from peripheral blood of a person infected with HIV-1 [Chen, B.K., et al., J. Virol., 68(2):654-660(1994)]. The cells were cultured in RPMI1640 medium containing 10 % FCS and 5 µM AZT.
(vi) MC141: This cell line was created by introducing HIV-LTR-SEAP reporter gene into HUT.78 cells by transfecting them with pSBR-HIV. This is a transfectant consistently expressing SEAP. The cells were cultured in RPMI1640 medium containing 10 % FCS and 300 µg/mL hygromycin.
(vii) CL-35: This cell line is a transfectant consistently expressing SEAP and was created by introducing HIV-LTR-SEAP reporter gene into U937 cells by transfecting them with pSBR-HIV. The cell were cultured in RPMI1640 medium containing 10 % FCS and 300 µg/mL hygromycin.
(viii) Clone#62: This was obtained by first isolating CD8-positive T cells from peripheral blood of an HIV-1 infected Japanese patient having a long lasting symptom-free history, through positive selection using magnetic beads coated with anti-CD8 antibody, and then immortalizing the obtained cells by bringing them into contact with an equal number of cells of an HTLV-1 producing T cell line, MT-2 (irradiated 100 rad), and finally, after one-month culture, cloning by means of limiting dilution. The supernatant of the culture of this clone exhibits potent SHIF (soluble, HIV reproduction inhibiting factor) activity. The cells were kept by passage in RPMI1640 medium containing 15 % FCS, 10 units/mL IL-2 and 10 % PBMC (human peripheral blood mononuclear cell) conditioned medium.
(ix) PBMC: This was obtained by purification of buffy coat from donated blood using Ficoll-Plaque (Amersham Pharmacia). Before use as a conditioned medium, this was cultured for thee days in RPMI1640 medium containing 3 µg/mL PHA, 1 unit/mL IL-2 and 10 % FCS and, for further six days after replacing the medium with the same one but free of PHA.

### <Preparation of Supernatant of CD8-positive Clone Culture>

Clone#62 that had been kept by passage in RPMI1640 medium was cultured for 3-4 days after replacement of its medium with PM1000 medium (Eiken Kagaku) containing 5 % FCS and 10 units/mL IL-2. One half of the medium was collected and cell culture was continued after addition of the same amount of the fresh medium. The collected supernatant was filtered through 0.22 µm filter to remove any precipitates and stored at -80°C.

### <Measurement of anti-HIV activity>

### 1) Co-culture Assay:

In a co-culture assay, a cell mixture is cultured consisting of cells from a cell line chronically infected with HIV and those from a non-infected cell line. This offers a testing system including all the stages of the viral life cycle, comprising virus adsorption on non-infected cells, infection, replication of the virus in the infected cells and release of the viral particle. Therefore, using such a co-culture system, anti-viral activity of a given test compound can be detected regardless of the stage on which the compound works, by measuring the amount of virus released from the cells and comparing the measured values between the two conditions, presence or absence of the test compound. In addition, anti-HIV activity of a test compound can be assessed for T cell-tropic HIV and macrophage-tropic HIV, respectively, by employing a combination of T cell lines (HUT.78 and T4/NL4-3) or macrophage lines (U1 and U937) as a combination of HIV chronically infected cells and non-infected cells.

Test Procedures: In a 48-well plate were placed 300 µL of a sample diluted with PM 1000 medium and 100 µL of RPMI 1640 medium containing 6 ng/mL TNF α and 20 % FCS. To this were added 100 µL of HUT.78 cells adjusted to 2 x 10⁵ cells/mL with OPTI-MEM I medium and 100 µL of T4/NL4-3 cells suspended in RPMI1640 medium at 5 x 10⁴ cells/mL (infected T4/NL4-3 : non-infected HUT.78 = 1:4) and the mixture was cultured for three days. One half of the culture supernatant was then replaced with fresh medium containing the same concentration of the sample and culture was continued for three more days. The supernatant of the six-day culture was collected and measured for the amount of the virus (p17) and the amount of HIV-LTR transcript (SEAP). For measurement, an HIV p17 antigen ELISA kit (Eiken Kagaku) and SEAP reporter gene assay chemiluminescent kit (ROCHE) were used according to the manufacturers' instructions. To the cells that had been cultured for six days was added 50 µL of MTS assay reagent (water soluble tetrazolium salt)(PROMEGA). The mixture was cultured for further four hours to allow color to develop, and the optical density measured at 490 nm, which was deemed to represent the number of living cells at the time they were subjected to measurement.

In the same manner, another co-culture system consisting of U 1 (chronically infected cell line) and U937 (non-infected cell line) (U1:U937 = 1:4) was also subjected to the assay.

### 2) Solo-culture Assay of Chronically Infected Cells:

In order to collect information on which stage of HIV life cycle is supressed by ATF as the basis of the ATF's observed overall anti-HIV activity, ATF was tested for anti-HIV activity in a solo-culture system consisting of chronically infected U 1 cells. As it was known that multiple infection with HIV would not occur in U 1 cells, any detected anti-HIV activity in the U1 cell solo-culture system would provide evidence that ATF acted at the stage of HIV provirus DNA transcription or later stages.

Test Procedures: In a 48-well plate was placed 300 µL of a sample diluted with PM1000 medium and 200 µL of RPMI1640 medium containing 6 ng/mL TNF α and 15 % FCS. To this was added 100 µL of chronically infected cells (U1) adjusted to 2 x 10⁵ cells/mL with OPTI-MEM I medium and the mixture was cultured for three days. One half of the culture supernatant was then replaced with fresh medium containing the same concentration of the sample and culture was continued for three more days. The supernatant of the six-day culture was collected and measured for the amount of the virus (p17).

### 3) Transient Transfection with Infectious HIV-DNA:

It is possible to artificially place infectious HIV-DNA onto the stage of its entering nucleus by forcibly introducing the viral DNA into the cell by means of liposomes. Using this system, it is possible to examine a given test compound for its suppressive activity at later stages of HIV life cycle than the penetration into the host cells.

Test Procedures: Four µg of infectious HIV-1 DNA (pNL4-3) and 10 µL of DMRIE-C reagent (GIBCO/BRL) were mixed. The mixture was used to transfect 2 x 10⁶ MC141 cells. Twenty-four hours later, the cells were collected and their density was adjusted to 2 x 10⁵ cells/mL with OPTI-MEM I medium. In a 48-well plate were placed 300 µL of a sample diluted with PM1000 medium and 200 µL of RPMI1640 medium containing 6 ng/mL TNFα and 15 % FCS. To this was added 100 µL of the transfected MC141 cells (2 x 10⁵ cells/mL ), and the mixture was cultured for three days. One half of the culture supernatant was then replaced with fresh medium containing the same concentration of the sample and culture was continued for four more days. The supernatant was collected eight days after transfection, and measured for the amount of the virus (p17) and the amount of HIV-LTR transcript (SEAP).

### 4) SEAP Reporter Assay in Non-infected Cells:

In HIV-non-infected cells (MC141 cells and CL35 cells) having incorporated HIV-LTR-SEAP reporter gene, stimulation with TNF α triggers activation of the HIV promoter LTR, leading to expression of the secretion-form alkaline phosphatase (SEAP) gene incorporated downstream of the promoter. These cells, therefore, can be used to examine whether a given test compound shows inhibitory activity at the stage of provirus transcription in the HIV life cycle, without involving actual reproduction of HIV. The degree of HIV-LTR transcription activity can be determined by measuring SEAP amount in the culture supernatant.

Test Procedures: In a 96-well plate was placed 50 µL of a sample diluted with serum-free PM1000 medium and 25 µL of RPMI1640 medium containing 6 ng/mL of TNFα and 20 % FCS. This then was inoculated with 25 µL of MC141 cells or CL35 cells prepared at the density of 2 x 10⁵ cells/mL with OPTI-MEM I medium (GIBCO/BRL). The cells were cultured with or without ATF, respectively, and the supernatant of 6-day culture, was collected and measured for SEAP amount contained in it using SEAP reporter gene assay chemiluminescent kit (ROCHE).

### 5) Assay of Inhibitory Activity on HIV Reproduction in Acute Infection (Transfection assay):

Four µg of infectious HIV-1 DNA (pNL4-3) and 10 µL of DMRIE-C reagent (GIBCO/BRL) were mixed and the mixture was used to transfect 2 x 10⁶ HUT.78 cells. Twenty-four hours later, the cells were collected, washed with OPTI-MEM I medium, and adjusted to the density of 2 x 10⁵ cells/mL. In a 48-well plate were placed 300 µL of PM1000 medium containing a stepwise-diluted sample or buffer solution and 200 µL of RPMI1640 medium containing 6 ng/mL of TNFα and 15 % FCS. To this was added 100 µL of the transfected HUT.78 cells (2 x 10⁵ cells/mL), and the mixture was cultured for four days. One half of the medium then was collected and replaced with fresh medium containing the same concentration of the sample or the buffer. The culture was continued for 12 days after infection, during which sampling and replacement of one half of the medium was repeated every four days.

Assay was performed in duplicate (n = 2), in which the virus amount in culture supernatant was measured using HIV p17 antigen ELISA kit (Eiken Kagaku).

### 6) Study of Effect of Anti-CD87 Antibody on Anti-HIV Activity of ATF

As ATF and HMW-uPA had been known to specifically bind to CD87 on the cell surface, it was expected that the anti-HIV activity observed both with ATF and HMW-uPA was mediated by their binding to CD87. To confirm this, a study was carried out to determine whether anti-CD87 antibody could block the anti-HIV activity of ATF.

Test Procedures: In a 48-well plate were placed 300 µL of anti-CD87 monoclonal antibody diluted with PM1000 medium ( # 3936 : AMERICAN DIAGNOSTICA INC.) and 100 µL of RPMI1640 medium containing 6 ng/mL TNFα and 20 % FCS. To this were added 100 µL of MC141 cells adjusted to the density of 2 x 10⁵ cells/mL with OPT-MEM I medium and 100 µL of T4/NL4-3 cells suspended at 5 x 10⁴ cells/mL in RPMI1640 medium, and the mixture was cultured for two hours (the final concentration of the antibody was 10 µg/mL). Two hours later, 12 µL of a sample containing ATF (final ATF concentration was approximately 3.3 ng/mL) was added. After three-day culture, one half of the culture supernatant was replaced with a fresh medium containing the same concentration of the antibody and the sample, and the culture was continued for further three days. The supernatant of the 6-day culture was collected and measured for the virus amount (p17) contained in it. As controls, similar culture was carried out using mediums not containing either or both of the antibody and ATF, respectively, and a medium containing non-specific IgG in place of the anti-CD87 antibody.

In the same manner, a study was also carried out with a co-culture system consisting of infected U1 and non-infected CL35 cells (U1:CL35 = 1:4).

### <Preparation of a Factor Having Anti-HIV Activity>

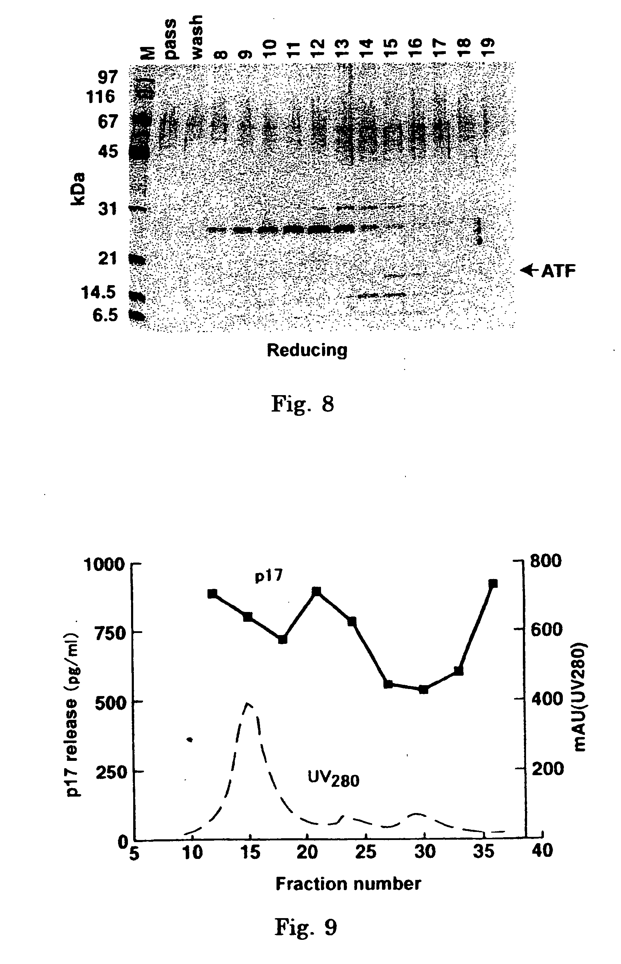

The whole process of purification below was carried out at 4°C unless otherwise mentioned.
1) First, 1 N hydrochloric acid was added to the supernatant to adjust the pH of the latter to 2.5 and the supernatant was left to stand for 24 hours at 4°C. With this treatment, potential risks of viral infection was eliminated and abundantly contained interferon γ was inactivated. One N sodium hydroxide solution then was added to adjust the pH of the mixture to 3.8. Five hundred mL of the pH-treated culture supernatant was loaded onto a SP Sepharose High Performance (AMERSHAM PHARMACIA) column (26 mm x 10 cm) that had been equilibrated with 25 mM acetate buffer (pH 3.8) containing 50 mM sodium chloride. After washing with 175 mL of the same buffer, the column was eluted with 175 mL of 50 mM HEPES/NaOH buffer (pH 7.4) (E1), and then with 175 mL of 50 mM HEPES/NaOH buffer (pH 7.4) containing 250 mM sodium chloride (E2). Each fraction was buffer-exchanged through a NAP-5 column and subjected to assay of its anti-HIV activity at 50 % concentration.
2) The activity was found collected in the E2 fraction from the SP Sepharose High Performance column. Sodium chloride was added to two lots of E2 fractions (corresponding to 1 L of the culture supernatant) up to the final concentration of 500 mM. The solution was loaded onto a Blue Sepharose 6FF (AMERSHAM PHARMACIA) column (26 mm x 10 cm) that had been equilibrated with 50 mM HEPES/NaOH buffer (pH 7.4) containing 500 mM sodium chloride. After washing with 175 mL of the same buffer, the column was eluted with 200 mL of 50 mM HEPES/NaOH buffer (pH 7.4) containing 1.8 M sodium chloride and 0.1 % CHAPS (E1). The fraction was buffer-exchanged through a NAP-5 column and subjected to assay of anti-HIV activity at 33 % concentration.
3) The activity was found to be collected in the E1 fraction from the Blue Sepharose 6FF column. The fraction was loaded onto a HiPreP Butyl 4FF (AMERSHAM PHARMACIA) column (16 mm x 10 cm) that had been equilibrated with 50 mM HEPES/NaOH buffer (pH 7.4) containing 1.8 M sodium chloride and 0.1 % CHAPS (P). After washing with 50 mL of the same buffer (W), the column was eluted with 100 mL of 0.1 % CHAPS/water (E). The fraction was buffer-exchanged through a NAP-5 column and subjected to assay of anti-HIV activity at 33 % concentration.
4) The activity was found collected in non-adsorbed fractions (P and W) from Butyl Sepharose column. Sodium chloride was added to three lots of the non-adsorbed fractions (corresponding to 3 L of the culture supernatant) up to the final concentration of 2 M. The solution was loaded onto a HiPreP Phenyl (HighSub) 6FF (AMERSHAM PHARMACIA) column (16 mm x 10 cm) that had been equilibrated with 50 mM HEPES/NaOH buffer (pH 7.4) containing 2 M sodium chloride and 0.1 % CHAPS. After washing with 175 mL of the same buffer, the column was eluted with 150 mL of 50 mM HEPES/NaOH buffer (pH 7.4) containing 250 mM sodium chloride and 0.1 % CHAPS (E1), and further with 100 mL of 0.1 % CHAPS/water (E2). The fractions were buffer exchanged through a NAP-5 column and subjected to assay of anti-HIV activity at 25 % concentration.
5) The activity was found collected in the E1 fraction. The fraction was loaded onto a hydroxyapatite (CHT-2, 20 µm; BioRad) column (10 mm x 10 cm) that had been equilibrated with 10 mM sodium phosphate buffer (pH 7.3) containing 0.1 % CHAPS. After washing with 50 mL of the same buffer (W), the column was eluted with 200 mM sodium phosphate buffer (pH 7.3) containing 0.1 % CHAPS (E200). The fraction was buffer-exchanged through a NAP-5 column and subjected to assay of anti-HIV activity at 25 % concentration.
6) The activity was found collected in the non-adsorbed fraction from the hydroxyapatite (P). The fraction was concentrated approximately forty-fold using a CentriPlus-10 (MW 10,000 cut) ultrafiltration membrane (AMICON MILLIPORE). The concentrated active fraction (3.75 mL) was loaded onto a HiPrep Sephacryl S-100 HR (AMERSHAM PHARMACIA) column (16 mm x 60 cm) that had been equilibrated with 10 mM sodium phosphate buffer (pH 6.4) containing 0.1 % CHAPS and 5 % glycerol. The column was eluted with 144 mL of the same buffer and the eluate was collected 2.5 mL each. The fractions were buffer exchanged through a NAP-5 column and subjected to assay of anti-HIV activity at 12.5 % concentration.
7) The active fractions were collected, then diluted two-fold with 10 mM sodium phosphate buffer (pH 6.4) containing 0.1 % CHAPS and loaded onto a Resource S (AMERSHAM PHARMACIA) column (0.64 x 3 cm) that had been equilibrated with the same buffer. After washing with 10 mL of the same buffer, the column was eluted with 10 mM sodium phosphate buffer (pH 6.4) containing 0.1 % CHAPS with a sodium chloride gradient of 0-500 mM (25 mL in total), and the eluate was collected 1 mL each. The fractions were subjected to assay of anti-HIV activity at 2.5 % concentration.
8) Active fractions were collected. Three lots of Resource S active fractions (corresponding to 9 L of the culture supernatant) were diluted 2.5-fold with 10 mM potassium phosphate buffer (pH 6.35) containing 0.1 % CHAPS and loaded onto a hydroxyapatite (CHT-2, 20 µm; BioRad) column (0.5 x 5 cm) that had been equilibrated with the same buffer. After washing with 5 mL of the same buffer, the column was eluted with a potassium phosphate gradient of 10 mM-400 mM (pH 6.35) (25 mL in total) containing 0.1 % CHAPS, and the eluate was collected 0.5 mL each.
   The fractions were subjected to assay of anti-HIV activity at 1 % concentration. For each fraction, determination of the anti-viral activity was performed using ELISA of p17 antigen released in the culture supernatant of a co-culture system consisting of an HIV chronically infected cell line and an non-infected cell line (1:4).
   In the cases where the sample concentration was 5 % or over, the sample was buffer-exchanged for serum-free PM1000 medium through a NAP-5 column (AMERSHAM PHARMACIA) that had been equilibrated with the medium in order to exclude any influence of the excess salt coming from the process of chromatography. Where the sample concentration was lower than 5 %, the sample was directly loaded, and fractions that had been obtained by a blank run of the chromatography were used as controls to eliminate any influence of the salt coming from the process of chromatography.
   The results are shown in Figs. 7 and 8. In Fig. 7, closed squares represent the anti-viral activity of the fractions assayed, the phantom line the UV absorption curve of the eluate, and the ascending line the concentration of potassium phosphate corresponding to each fraction, respectively. Fig. 8 shows SDS/PAGE electrophoresis (reductive condition) of fractions 8-19. As seen in Figs. 7 and 8, bands of about 18 kDa were detected in fractions exhibiting anti-HIV activity as determined in terms of the inhibition of p17 release.
9) Active fractions were collected and concentrated 15-fold using a Centricon-10 (MW 10,000 cut) ultrafiltration membrane (MILLIPORE). To the concentrate of two lots of active fractions from the hydroxyapatite column (corresponding to 18 L of the culture supernatant), trifluoroacetic acid (TFA) was added to make a final concentration of 0.2 % and the mixture was loaded onto a Resource RPC column (AMERSHAM PHARMACIA) column (0.64 x 3 cm) that had been equilibrated with 0.2 % trifluoroacetic acid/water. After washing with 5 mL of the buffer, the column was eluted with 5 mL of eluant with a gradient of up to 0.2 % TFA/30 % acetonitrile, then with 15 mL of eluant with a gradient of up to 0.2 % TFA/50 % acetonitrile, and finally with 5 mL of eluant with a gradient of up to 0.2 % TFA/100 % acetonitrile, and the eluate was collected 0.5 mL each. This reverse-phase column chromatography was carried out at 10°C. The anti-HIV activity was determined at 0.2 % concentration. As a result, bands of 18 kDa were also detected on SDS/PAGE in the respective eluate fractions from this Resource RPC column that exhibited anti-HIV activity.

The active fractions, which were collected and dried under reduced pressure, gave about 0.9 µg (500 pmol) of the 18 kDa protein. For activity assay, this protein was dissolved in a phosphate buffer (PBS) containing 0.5 % BSA and 0.1 % CHAPS. For sequencing, the protein was dissolved in a SDS/PAGE sample buffer.

### <Amino Acid Sequencing>

A portion of the 18 kDa protein purified above was subjected to SDS/PAGE, stained with Coomassie Brilliant Blue (CBB), and corresponding bands was cut out. The cut out piece of the gel was directly trypsinized and subjected to peptide mapping. Three of the obtained peaks were analyzed for their amino acid sequence on a sequencer. As a result, the presence of the following inner sequences was found.
·the amino acid sequence set forth as SEQ ID NO:3 (Fragment 1)
·the amino acid sequence set forth as SEQ ID NO:4 (Fragment 2)
·the amino acid sequence set forth as SEQ ID NO:5 (Fragment 3)

The amino acid sequences of Fragments 1, 2 and 3 matched with the sequence of the amino-terminal fragment (ATF, also called "long A chain") of urokinase-type plasminogen activator. The results of peptide mapping and amino acid sequencing revealed that the protein purified above was ATF.

### <Purification of ATF from Urokinase Bulk Material>

In the active fraction, no band was detected corresponding to the single-chain urokinase-type plasminogen activator, the HMW-uPA, or the LMW-uPA. This strongly suggested that ATF exhibited the anti-HIV activity. The present inventors were then prepared ATF from human urine urokinase bulk material and assessed its anti-HIV activity as described below.

A 4.5-mL aliquot of human urine urokinase bulk material (JUN-9604) (containing approximately 50,000-unit urokinase) produced at Seishin Factory of JCR Pharmaceuticals Co., Ltd. was loaded onto a HiPrep Sephacryl S-100 column (16 mm x 60 cm) that had been equilibrated with 10 mM sodium phosphate buffer (pH 6.4) containing 0.1 % CHAPS and 100 mM sodium chloride. The column was eluted with 144 mL of the same buffer and the eluate was collected 2.5 mL each. The activity was measured at 1 % concentration.

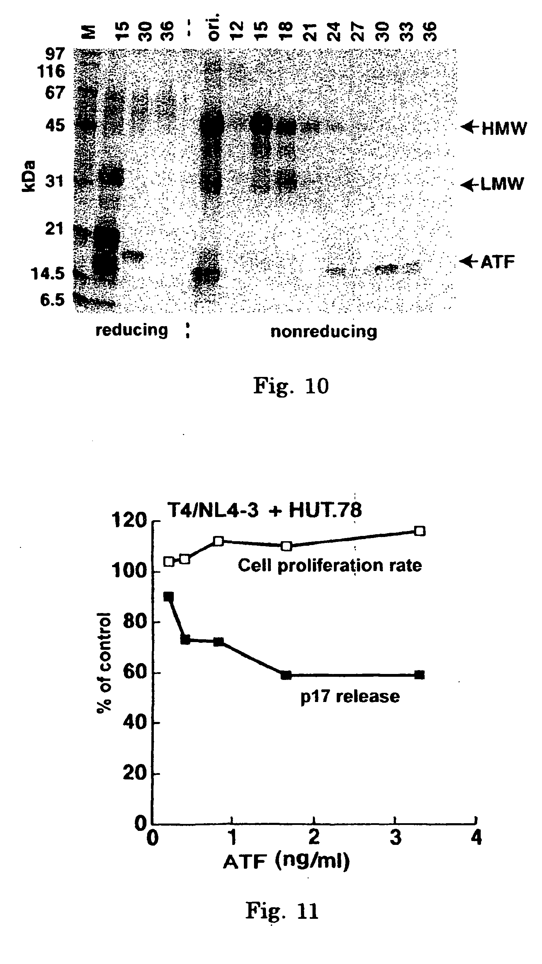

As a result of the analysis, it was found that the above urokinase bulk material comprised HMW-uPA, LMW-uPA and ATF, at a proportion of about 9:1:1. Among the fractions through the HiPrep Sephacryl S-100 column, those containing only ATF (Nos. 27-33) exhibited potent anti-HIV activity (inhibition of p17 release) (Figs. 9 and 10). This indicates that ATF has an anti-HIV activity, as was expected from the previous test performed with soluble HIV reproduction-suppressing factor purified from clone#62 supernatant.

In addition to the anti-HIV activity of ATF, the fractions containing HMW-uPA (Nos. 15-18) also were found to have anti-HIV activity (p17), thought that was weaker than ATF (Figs. 9 and 10).

### <Results of Assay of ATF's Anti-HIV Activity in Co-culture systems>

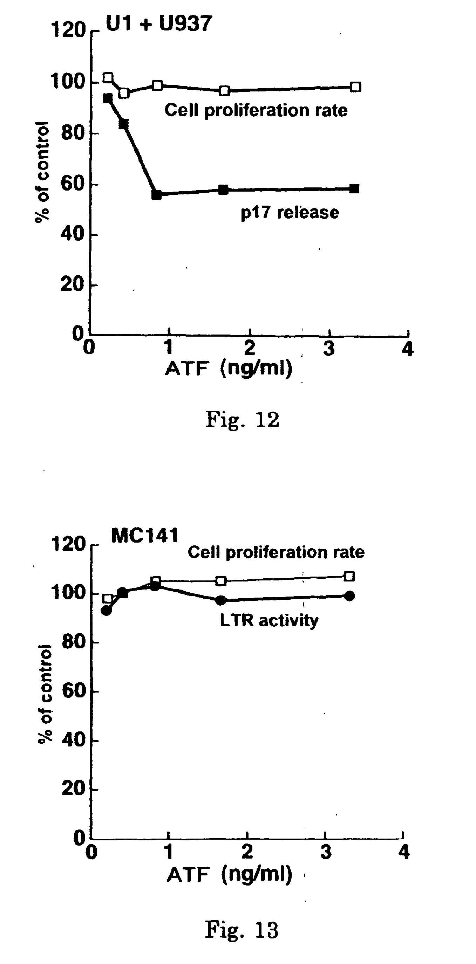

The purified ATF concentration-dependently lowered the amount of p17 appearing in the supernatant of the co-culture consisting either of the T cell lines or the macrophage lines. The rate of inhibition at the concentration of 1.5 ng/mL was about 40 % (Figs. 11 and 12). As the addition of ATF did not alter the rate of proliferation of the cultured cells as compared with the control (Figs. 11 and 12), it was clear that ATF did not affect cell proliferation and was not cytotoxic. These findings indicate that the anti-HIV activity observed with ATF is not due to some kind of cytotoxicity, that ATF exhibits anti-HIV activity at very low concentrations, and that ATF exhibits substantially comparable anti-HIV activity against both of T cell-tropic HIV and macrophage-tropic HIV.

### <Results of SEAP Reporter Assay in Culture of Non-infected Cells>

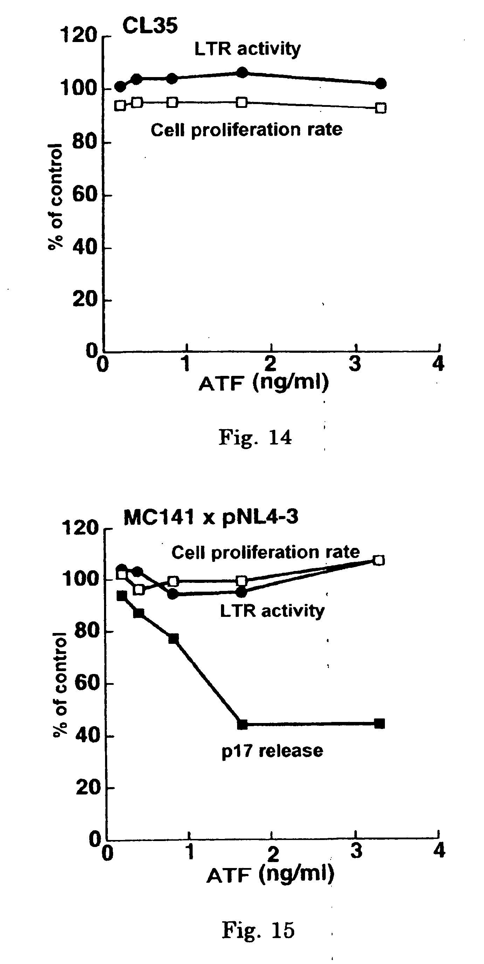

See Figs. 13 and 14. In the SEAP reporter assay in the solo-culture of MC141 and CL35 cells, ATF did not affect the amount of alkaline phosphatase in the culture supernatant of either type of the cells (Figs. 13 and 14). This indicates that ATF does not inhibit HIV reproduction at the stages of HIV-LTR-promoted transcription or translation that follows to it. Under the conditions described, it was also confirmed that ATF did not affect the rate of proliferation of the cells (Figs. 13 and 14).

### <Results of Temporary Transfection with Infectious HIV-DNA>

See Fig. 15. Also in the assay using MC141 cells temporarily transfected with infectious HIV-DNA (pNL4-3), 1.5 ng/mL ATF suppressed the release of HIV p17 into the culture supernatant by about 60 %. However, ATF had no effect on SEAP expression, therefore on transcription promoting activity of LTR. No influence was observed on the rate of proliferation of the cells, either.

The fact that the release of p17 was inhibited by ATF while the level of LTR-promoted transcription was not affected by ATF, indicates that ATF's observed anti-HIV activity was not resulting from any inhibition at the stages of LTR-promoted transcription, HIV-tat-enhanced transcription or subsequent translation, and suggests that the observed anti-HIV activity was the result of inhibition at some later stages following translation, i.e., HIV particle assembling or budding.

### <Results of Solo-culture Assay of Chronically Infected Cells>

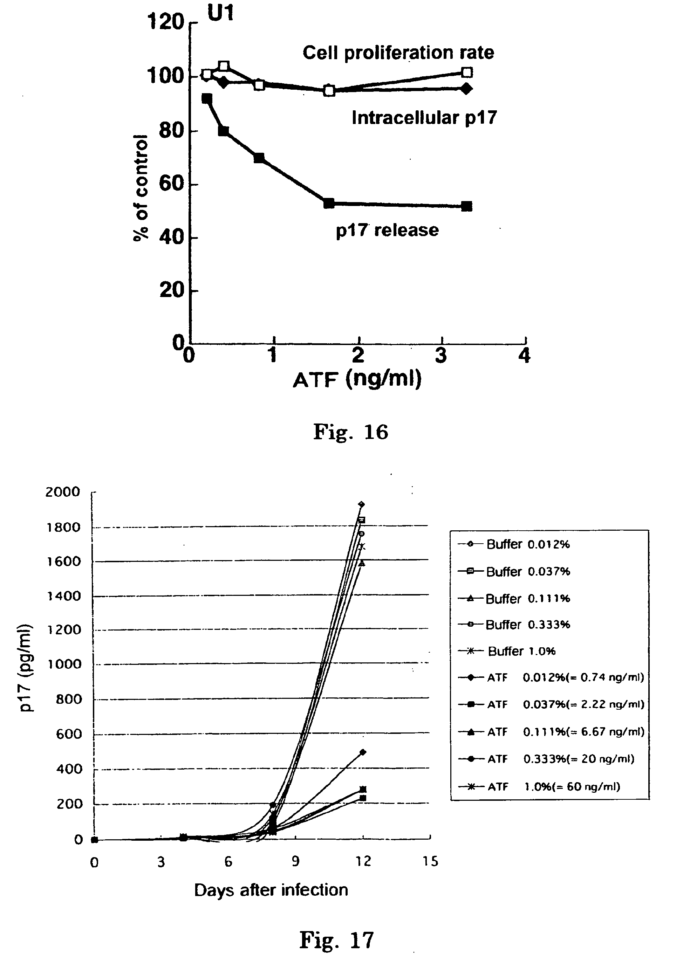

See Fig. 16. In the solo-culture assay of chronically infected cells (U1), 1.5 ng/mL ATF inhibited the release into the culture supernatant of the viral antigen p17 by about 50 %. However, intracellular p17 was not affected by ATF. ATF did not affect the rate of proliferation of the cells, either.

As it is known that multiple infection with HIV will not take place in U1 cells, anti-HIV activity detectable in U1 cell solo culture is limited to such an activity that works at the stage of transcription of proviral DNA or later stages. Therefore, the suppression by ATF of the release of viral particles (p17) in the U1 cell solo culture indicates that ATF has suppressive activity at the stage of transcription of proviral DNA or later stages. On the other hand, as ATF did not affect the amount of intracellular p17, it is apparent that ATF does not affect any of the stages from transcription of HIV provirus DNA to translation of HIV mRNA. This result from the solo-culture assay of the chronically infected cells is the same as the result obtained from the aforementioned temporary transfection assay. These findings strongly suggest that ATF suppresses some later stages after translation of HIV mRNA, i.e., stages from HIV particle assembling to budding of viral particles.

### <Result of Assay of Inhibitory Activity on Reproduction of HIV in Acute Infection (Transfection Assay)>

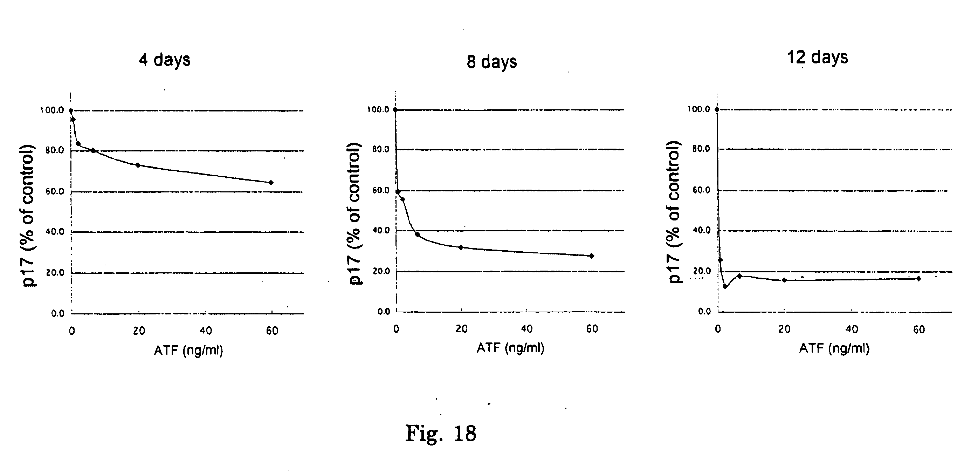

See Fig. 17, which illustrates the profile of virus amount over the days following the start of the culture. It is seen in the figure that, in the control group, the amount of the virus rapidly increased during the period from the 8th to 12th days after the start of the culture. There was found little influence of the buffers used. In ATF groups, on the other hand, the rate of virus reproduction was markedly reduced, and the suppression of viral reproduction was found dependent on ATF concentrations. Furthermore, the rate of suppression of virus reproduction increased with the lapse of culture days (see Fig. 18), showing more than 75 % at 0.74 ng/mL ATF, and more than 87 % at 2.22 ng/mL ATF, respectively, after 12 days of infection.

### <Effect of anti-CD87 Antibody on Anti-HIV Activity of ATF>

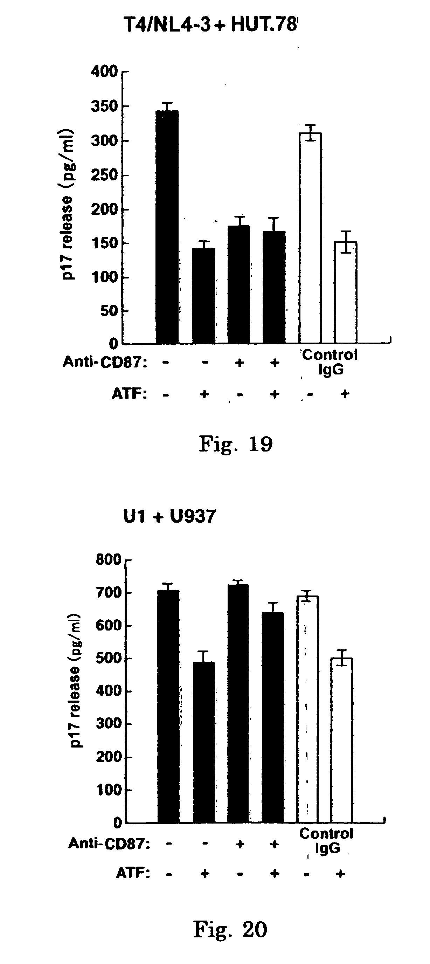

See Fig. 19. In the co-culture system of T cell lines, T4/NL4-3 and MC141, nearly equal suppression of p17 release into the culture supernatant was observed irrespective of the medium used, i.e., a medium containing 3.3 ng/mL ATF only or a medium containing both of 3.3 ng/mL ATF and 10 µg/mL monoclonal antibody to CD87, which is the receptor for ATF and HMW-uPA. On the other hand, 10 µ g/mL anti-CD87 antibody itself was found to anti-HIV activity have largely comparable to 3.3 ng/mL ATF. As comparable levels of suppression of p17 release were observed with mediums containing anti-CD87 antibody or ATF or both of them, it is considered that both ATF and anti-CD87 antibody act through a common target molecule on the cell. In addition, the culture performed in the presence of non-specific IgG in place of anti-CD87 antibody gave the same result as the result obtained from the culture without any antibody. This finding indicates that the observed anti-HIV activity of anti-CD87 antibody depends on its specificity. Therefore, the antibody is considered to have suppressed HIV release through specific binding to CD87 on the cell. That the addition of ATF to the medium containing anti-CD87 antibody caused no enhancement of suppression of HIV release is considered to be due to ATF being kept from binding to CD87, which had already been blocked by anti-CD87 antibody. More importantly, since anti-CD87 antibody and ATF both exhibit anti-HIV activity (p17) in the T cell lines and both are compounds specifically binding to CD87, the test results obtained above indicate that specific binding of CD87 to its ligand molecules causes, by some unknown mechanisms in the cell, suppression of HIV release (at the stage of assembling or budding).

On the other hand, in the co-culture system consisting of macrophage lines U1 and U937 (see Fig. 20), addition of anti-CD87 antibody blocked the anti-HIV activity of ATF almost completely. In addition, anti-CD87 antibody did not show anti-HIV activity in these cell lines. These results, therefore, differ from the above results obtained with T cell lines. However, this discrepancy can be explained as follows; anti-CD87 antibody did not exhibit anti-HIV activity on macrophages for some reason and acted simply to block CD87 and thereby prevented ATF from binding to CD87 in a cell culture in a medium containing both ATF and anti-CD87 antibody. Therefore, the previous conclusion that ATF suppresses HIV reproduction via binding to CD87 is also supported by the fact that ATF's anti-HIV activity was blocked by anti-CD87 antibody in the macrophage lines.

CD87 as well as its complex is localized on a sphingolipid-rich cell surface structure called "lipid raft" [Koshelnic, Y. et a., Thromb. Haemost., 82(2):305-311(1999)], while budding of HIV is reported to take place selectively in the region of lipid raft [Nguyen, D.H. et al., J. Virol., 74(7):3264-3272(2000)]. In addition, it is known that Thy-1, which is also localized in the region of lipid raft, is selectively taken up by HIV in its envelope [Nguyen, D.H. et al., J. Virol., 74(7):3264-3272(2000)]. Put together, these reports and the findings from the experiments by the present inventors suggest a probable mechanism that ligands molecules to CD87, such as ATF, inhibit budding of HIV by acting, via CD87, on component molecules of the lipid raft.

### <Preparation Example 1> Preparation for Intravenous, Subcutaneous or Intramuscular Injection .

According to the following formula, necessary amount of the components are mixed to form a solution, and the solution is filter-sterilized through a membrane filter with the pore size of 0.22 µm to make an intended preparation.

| | |
|---|---|
| ATF | 10 mg |
| Mannitol | 50 mg |
| Distilled water | to 1 mL |

### <Preparation Example 2> Preparation for Intravenous, Subcutaneous or Intramuscular Injection

According to the following formula, necessary amount of the base components are mixed to form a solution. After addition of ATF, the solution is made to volume and filter-sterilized through a membrane filter with the pore size of 0.22 µm to make an intended preparation.

| | |
|---|---|
| ATF | 50 mg |
| Sodium chloride | 8.6 mg |
| Potassium chloride | 0.3 mg |
| Calcium chloride | 0.33 mg |
| Distilled water for injection | to 1 mL |

### <Preparation Example 3> Preparation for Intravenous, Subcutaneous or Intramuscular Injection

According to the following formula, necessary amount of the base components are mixed to form a solution. After addition of ATF, the solution is made to volume and filter-sterilized through a membrane filter with the pore size of 0.22 µm to make an intended preparation.

| | |
|---|---|
| ATF | 50 mg |
| Sodium chloride | 8.3 mg |
| Potassium chloride | 0.3 mg |
| Calcium chloride | 0.33 mg |
| Sodium hydrogen phosphate. 12H₂O | 1.8 mg |
| IN hydrochloric acid | q.s. (pH 7.4) |
| Distilled water for injection | to 1 mL |

### <Preparation Example 4> Preparation for Intravenous, Subcutaneous or Intramuscular Injection

According to the following formula, necessary amount of the base components are mixed to form a solution. After addition of ATF, the solution is made to volume and filter-sterilized through a membrane filter with the pore size of 0.22 µm to make an intended preparation.

| | |
|---|---|
| ATF | 50 mg |
| Sodium chloride | 8.3 mg |
| Potassium chloride | 0.3 mg |
| Calcium chloride | 0.33 mg |
| Glucose | 0.4 mg |
| Sodium hydrogen phosphate 12H₂O | 1.8 mg |
| 1N hydrochloric acid | q.s. (pH 7.4) |
| Distilled water for injection | to 1 mL |

### <Preparation Example 5> Preparation for Pulmonary Administration

According to the following formula, ATF and lactose are weighed and dissolved in 120 mL of purified water to provide a spray solution, and subjected to spray drying by a conventional method to form a preparation for pulmonary administration.

| | |
|---|---|
| ATF | 100 mg |
| Lactose (monohydrate) | 2900 mg |
| Total | 3000 mg |

### <Preparation Example 6> Preparation for Pulmonary Administration

According to the following formula, ATF and hydroxypropylcellulose are weighed and dissolved in 120 mL of purified water to provide a spray solution, and subjected to spray drying by a conventional method to form a preparation for pulmonary administration.

| | |
|---|---|
| ATF | 100 mg |
| Hydroxypropylcellulose | 2900 mg |
| Total | 3000 mg |

### <Preparation Example 7> Preparation for Pulmonary Administration

According to the following formula, ATF and hydrogenated lecithin are weighed and dissolved in 120 mL of purified water to provide a spray solution, and subjected to spray drying by a conventional method to form a preparation for pulmonary administration.

| | |
|---|---|
| ATF | 100 mg |
| Hydrogenated lecithin | 2900 mg |
| Total | 3000 mg |

### <Preparation Example 8> Preparation for Pulmonary Administration

According to the following formula, ATF, hydroxypropylcellulose and D-mannitol are weighed and dissolved in 90 mL of purified water to provide a spray solution, and subjected to spray drying by a conventional method to form a preparation for pulmonary administration.

| | |
|---|---|
| ATF | 240 mg |
| Hydroxypropylcellulose | 129 mg |
| D-mannitol | 2631 mg |
| Total | 3000 mg |

The present invention provides a novel type of anti-HIV agent that suppresses HIV reproduction in an infected patient by a different mechanism of action from those known with conventional drugs. Thus, the present invention serves to widen a choice of AIDS therapeutic means aiming for both prophylaxis before, and treatment after, the onset of the disease, thereby improving efficacy of AIDS therapies in combination with conventional anti-HIV drugs.

## Claims

1. A method for screening for an anti-HIV agent comprising separately bringing compounds to be tested into contact with CD87 and selecting from the compounds a compound that specifically binds to CD87.

2. Use of a ligand molecule binding to CD87 for the manufacture of a pharmaceutical composition for suppression of reproduction of HIV in a human infected with HIV wherein the ligand molecule binding to CD87 is a fragment of the high molecular weight urokinase-type plasminogen activator, wherein the fragment comprises amino acids 21-155 of the prepro-urokinase (sc-uPA) and does not extend beyond amino acid 178 of the sc-uPA.

3. The use of claim 2 wherein the ligand molecule binding to CD87 is ATF.

4. The use of claim 2 wherein the ligand molecule binding to CD87 is a fragment of to ATF, wherein the fragment has a specific binding affinity to CD87.

5. Use of a ligand molecule binding to CD87 for the manufacture of a pharmaceutical composition for suppression of reproduction of HIV in a human infected with HIV wherein the ligand molecule binding to CD87 is an anti-CD87 antibody.

6. The use of claim 5 wherein the ligand molecule binding to CD87 is a fragment of an anti-CD87 antibody, wherein the fragment has a specific binding affinity to CD87.

## Patentansprüche

1. Verfahren zum Screening nach einem Anti-HIV-Mittel, umfassend das getrennte Inkontaktbringen von Verbindungen, die getestet werden sollen, mit CD87 und Auswählen einer Verbindung, die spezifisch an CD87 bindet, aus den Verbindungen.

2. Verwendung eines Ligandenmoleküls, das an CD87 bindet, zur Herstellung einer pharmazeutischen Zusammensetzung zum Unterdrücken der Reproduktion von HIV in einem Menschen, der mit HIV infiziert ist, wobei das Ligandenmolekül, das an CD87 bindet, ein Fragment des Hochmolekulargewichts-Urokinasetyp Plasminogenaktivators ist, wobei das Fragment die Aminosäuren 21 bis 155 der Preprokinase (sc-uPA) umfasst und nicht über Aminosäure 178 des sc-uPA hinausgeht.

3. Verwendung gemäß Anspruch 2, wobei das Ligandenmolekül, das an CD87 bindet, ATF ist.

4. Verwendung gemäß Anspruch 2, wobei das Ligandenmolekül, das an CD87 bindet, ein Fragment des ATF ist, wobei das Fragment eine spezifische Bindungsaffinität an CD87 besitzt.

5. Verwendung eines Ligandenmoleküls, das an CD87 bindet, zur Herstellung einer pharmazeutischen Zusammensetzung zum Unterdrücken der Reproduktion von HIV in einem Menschen, der mit HIV infiziert ist, wobei das Ligandenmolekül, das an CD87 bindet, ein Anti-CD87-Antikörper ist.

6. Verwendung gemäß Anspruch 5, wobei das Ligandenmolekül, das an CD87 bindet, ein Fragment eines Anti-CD87-Antikörpers ist, wobei das Fragment eine spezifische Bindungsaffinität an CD87 besitzt.

## Revendications

1. Procédé pour cribler pour un agent anti-VIH, consistant à porter séparément les composés devant être testés en contact avec CD87 et à sélectionner, parmi les composés, un composé qui se lie spécifiquement à CD87.

2. Utilisation d'une molécule de ligand se liant à CD87, pour la fabrication d'une composition pharmaceutique destinée à supprimer la reproduction du VIH chez un humain infecté par le VIH, dans laquelle la molécule de ligand se liant à CD87 est un fragment de l'activateur du plasminogène de type urokinase de poids moléculaire élevé, dans laquelle le fragment comprend les acides aminés 21 à 155 de la prépro-urokinase (sc-uPA) et ne s'étend pas au-delà de l'acide aminé 178 de la sc-uPA.

3. Utilisation selon la revendication 2, dans laquelle la molécule de ligand se liant à CD87 est l'ATF.

4. Utilisation selon la revendication 2, dans laquelle la molécule de ligand se liant à CD87 est un fragment d'ATF, dans laquelle le fragment a une affinité de liaison spécifique pour CD87.

5. Utilisation d'une molécule de ligand se liant à CD87, pour la fabrication d'une composition pharmaceutique destinée à supprimer la reproduction du VIH chez un humain infecté par le VIH, dans laquelle la molécule de ligand se liant à CD87 est un anticorps anti-CD87.

6. Utilisation selon la revendication 5, dans laquelle la molécule de ligand se liant à CD87 est un fragment d'un anticorps anti-CD87, dans laquelle le fragment a une affinité de liaison spécifique pour CD87.
